# EUROPEAN PATENT APPLICATION

(11) **EP 2 554 107 A1**
(43) Date of publication of application: **06.02.2013**
(21) Application number: 12178708.9
(22) Date of filing: 31.07.2012
(51) Int. Cl.: A61B 1/24, A61B 1/06, A61B 1/04, A61B 5/00

(54) **Intraoral inspection apparatus and method for operating intraoral inspection apparatus**

(30) Priority: 05.08.2011 JP 2011171763
(71) Applicant: GC Corporation, Bunkyo-ku Tokyo 113-0033 (JP)
(72) Inventor: Walsh, Laurence J., Brisbane Queensland 4000 (AU); Takada, Mitsuaki, Itabashi-Ku Tokyo 174-8585 (JP); Shinjo, Takao, Itabashi-Ku Tokyo 174-8585 (JP)
(74) Representative: Smart, Peter John

(57) **Abstract**

The present invention provides an intraoral inspection apparatus for dental use which is capable of distinguishing and detecting more kinds of diseased sites and the like with one apparatus.

The intraoral inspection apparatus comprises: a light source unit 11 which is provided with color-producing light sources 14 capable of inducing fluorescent lights from diseased sites by irradiating an oral cavity; a wavelength adjusting device 16 which is provided with a first filter 19 that transmits, among the fluorescent lights, a light having a wavelength of a fluorescent light from a predetermined diseased site and blocks or attenuates a light having a wavelength other than this wavelength, and provided with a second filter 20 that transmits, among the fluorescent lights, a light having a wavelength of a fluorescent light from a diseased site different from the predetermined diseased site and blocks or attenuates a light having a wavelength other than this wavelength; and an imaging device 21 which receives the fluorescent light transmitted through the first filter and the fluorescent light transmitted through the second filter, and converts them into electrical signals.

## Description

### Technical Field

The present invention relates to an intraoral inspection apparatus for dental use, and a method for operating an intraoral inspection apparatus. More particularly, it relates to an intraoral inspection apparatus and a method for operating an intraoral inspection apparatus which make it easy to distinguish a disease, treated site, normal site, and the like in the oral cavity.

### Background Art

In the oral cavity, there are various conditions which soft tissue and hard tissue are affected, such as sites with various types of diseases, already treated sites, and normal sites ( which may be collectively referred to as "diseased sites and the like" hereinafter). In order to distinguish and recognize all of these diseased sites and the like with the naked eye, specialized knowledge and experience by a dental clinician are required. Even for a practitioner with such knowledge and experience, it is convenient to be able to distinguish the diseased sites and the like more easily. Moreover, patients who are shown a conventional photographic or video image of their own oral cavity cannot easily recognize the existence of the diseased sites and the like; therefore, with the conventional images, dentists often face a difficulty in explaining the conditions of the oral cavity to their patient clearly.

It is known that when irradiating soft tissue and hard tissue in the oral cavity with a light having a peak value within a predetermined wavelength range, a periodontally-diseased site in the oral cavity and a resin filled in the tooth emit fluorescence having a wavelength longer than a wavelength of the irradiated light. The "QLF method (Quantitative Light-Induced Fluorescence, which may be referred to as "QLF" hereinafter)" is known as a method for quantitatively evaluating diseased sites and the like using the above characteristic.

For example, Patent Document 1 discloses a dental inspection apparatus for inspecting diseased sites and the like quantitatively by QLF. In addition, Patent Document 2 discloses a dental appliance for inspecting tooth surfaces by employing QLF for the purpose of identifying diseased sites.

### Citation List

### Patent Documents

Patent Document 1: Japanese Patent Application Laid-Open (JP-A) No. 2006-174977
Patent Document 2: JP-A No. 2004-65994

### Summary of the Invention

### Problems to be Solved by the Invention

However, the dental inspection apparatus and dental appliance mentioned above only allow a dentist to distinguish a few diseased sites and the like clearly, or they require complex processing in identifying the diseased sites and the type of the disease. As such, it is very beneficial to be able to detect a plurality of diseases with one apparatus or appliance.

In view of the above problems, an object of the present invention is to provide an intraoral inspection apparatus for dental use which is capable of easily detecting more kinds of diseased sites and the like with one apparatus, and to provide a method for operating an intraoral inspection apparatus.

### Means for Solving the Problem

The present invention will be described below. In order to make the description easy to understand, reference numerals in the accompanying drawings are represented in parentheses; however, the present invention is not limited to an embodiment illustrated in the drawings.

A first aspect of the present invention is an intraoral inspection apparatus (10) comprising: a light source unit (11) which is provided with color-producing light sources (14, 15) capable of inducing fluorescent lights from diseased sites by irradiating an oral cavity; a wavelength adjusting device (16) which is provided with a first filter (19) that transmits, among the fluorescent lights, a light having a wavelength of a fluorescent light from a predetermined diseased site and blocks or attenuates a light having a wavelength other than this wavelength, and provided with a second filter (20) that transmits, among the fluorescent lights, a light having a wavelength of a fluorescent light from a diseased site different from the predetermined diseased site and blocks or attenuates a light having a wavelength other than this wavelength; and an imaging device (21) which receives the fluorescent light transmitted through the first filter and the fluorescent light transmitted through the second filter, and converts them into electrical signals.

A second aspect of the present invention is the intraoral inspection apparatus (10) according to the first aspect, further comprising a white light source (13).

A third aspect of the present invention is the intraoral inspection apparatus (10) according to the first or second aspect, wherein a first QLF mode and a second QLF mode can be switched automatically or manually, the first QLF mode being a mode in which the color-producing light sources (14, 15) are turned on and the first filter (19) is disposed such that the light transmitted through the first filter is received by the imaging device (21), and the second QLF mode being a mode in which the color-producing light sources are turned on and the second filter (20) is disposed such that the light transmitted through the second filter is received by the imaging device.

A fourth aspect of the present invention is a method for operating an intraoral inspection apparatus, wherein the intraoral inspection apparatus (10) according to any one of the first to third aspects is used; the color-producing light source (14) is turned on; a light from the color-producing light source (14) is irradiated into the oral cavity; the light from the oral cavity is received by the imaging device (21) through the first filter (19); the color-producing light source (15) is turned on; a light from the color-producing light source is irradiated into the oral cavity; and the light from the oral cavity is received by the imaging device through the second filter (20).

A fifth aspect of the present invention is a method for operating an intraoral inspection apparatus, wherein the intraoral inspection apparatus according to the second aspect is used; the color-producing light source (14) is turned on; a light from the color-producing light source is irradiated into the oral cavity; the light from the oral cavity is received by the imaging device (21) through the first filter (19); the color-producing light source (15) is turned on; a light from the color-producing light source is irradiated into the oral cavity; the light from the oral cavity is received by the imaging device through the second filter (20); the white light source is turned on; and a light from the oral cavity is received by the imaging device.

### Effects of the Invention

According to the present invention, it is possible to detect a plurality of diseased sites and the like of more kinds than before with one intraoral inspection apparatus.

### Brief Description of the Drawings

Fig. 1 is a conceptual view of an intraoral inspection apparatus according to one embodiment;
Fig. 2 is a view of the intraoral inspection apparatus of Fig. 1 seen from the right side of Fig. 1; and
Fig. 3 is a perspective view of a wavelength adjusting device.

### Description of Modes for Carrying Out the Invention

The functions and benefits of the present invention described above will be apparent from the following modes for carrying out the invention. Hereinafter, the present invention will be described based on the embodiment illustrated in the drawings. However, the invention is not limited to the embodiment.

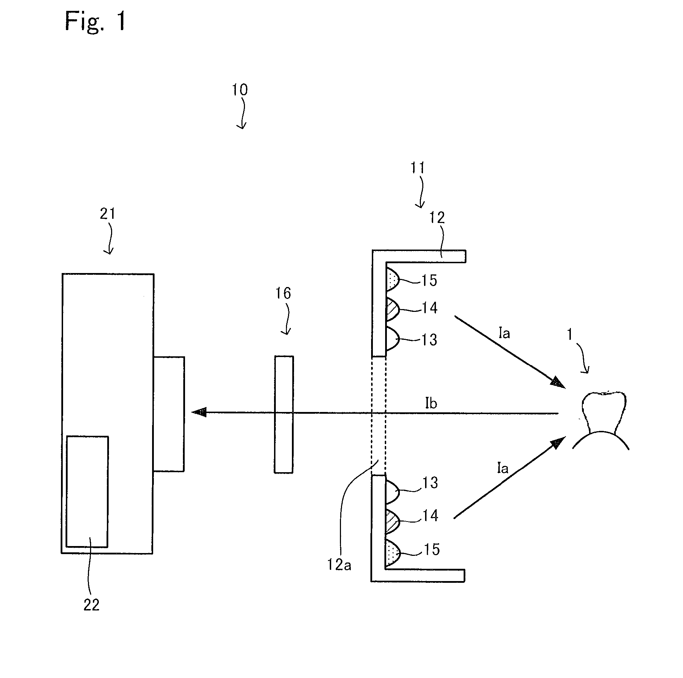

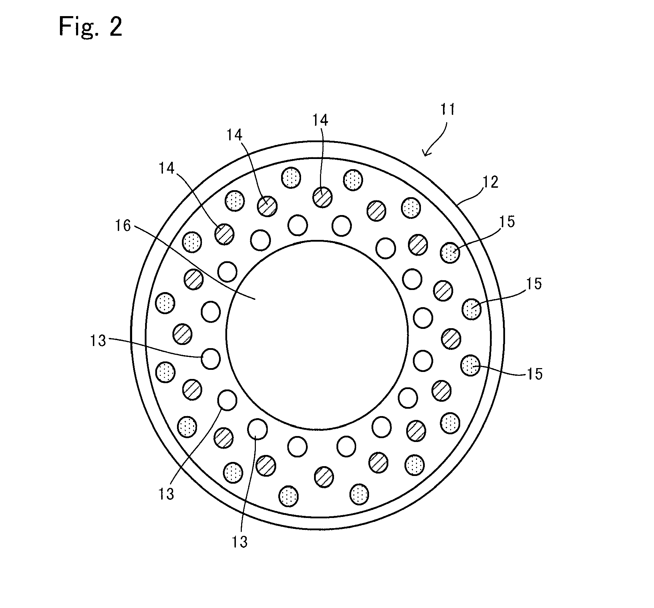

Fig. 1 is a view conceptually illustrating an intraoral inspection apparatus 10 according to one embodiment. Fig. 2 is a view of the intraoral inspection apparatus 10 of Fig. 1 seen from the right side of Fig. 1.
The intraoral inspection apparatus 10 comprises: a light source unit 11; a wavelength adjusting device 16; an imaging device 21; and a processing device 22.

As seen from Figs. 1 and 2, the light source unit 11 comprises: a hood 12; a white light source 13; a first color-producing light source 14; and a second color-producing light source 15.
The hood 12 is a cylindrical member with a bottom plate, and a circular hole 12a is formed on the bottom plate, having an axis center of the cylindrical hood 12 in the center. A light reaches the below-described wavelength adjusting device 16 and imaging device 21 through the hole 12a. The hood 12 functions as a so-called light-guiding means for efficiently guiding, into the oral cavity, the lights emitted from the white light source 13, the first color-producing light source 14, and the second color-producing light source 15. In addition to this, the hood 12 can prevent the lights emitted from the white light source 13, the first color-producing light source 14, and the second color-producing light source 15 from directly entering the eyes of a practitioner and a patient.
The inner surface of the hood 12 preferably has a high light reflectivity. This enables the lights emitted from the light sources to be efficiently guided into the oral cavity, and can enhance the function of the hood as a light-guiding means. An example of the surface having a high light reflectivity may be a mirror surface.

The white light source 13 is a light source which emits a light having a wavelength ranging over the entire visible light region. As the white light source, a light source conventionally used for the observation of the oral cavity may be used. Accordingly, although the type of the white light source 13 is not particularly limited, the white light source 13 is preferably a white light-emitting diode (white LED) in view of life, brightness, and power consumption of the light source.

The first color-producing light source 14 is a first color-producing light source for irradiating the oral cavity with a light having a specific wavelength. This specific wavelength of the first color-producing light source 14 can be adequately selected without particular limitations as long as the wavelength is within a range suitable for extracting information on a disease in relation to the filter provided to the below-described wavelength adjusting device 16. The type of the light source is not particularly limited; however, in view of life of the light source, brightness of the light emitted from the light source, and power consumption of the light source, a light-emitting diode (LED) having a peak wavelength within this wavelength range is preferable.
As described below, the first color-producing light source 14 enables detection of various types of diseased sites and the like in relation to the filter provided to the wavelength adjusting device 16.

The second color-producing light source 15 is a second color-producing light source for irradiating the oral cavity with a light having a wavelength different from the wavelength of the above first color-producing light source 14. This specific wavelength of the second color-producing light source 15 can be adequately selected without particular limitations as long as the wavelength is within a range suitable for extracting information on a disease different from the disease detected using the first color-producing light source 14, in relation to the filter provided to the below-described wavelength adjusting device 16. The type of the light source is not particularly limited; however, in view of life of the light source, brightness of the light emitted from the light source, and power consumption of the light source, a light-emitting diode (LED) having a peak wavelength within this wavelength range is preferable.
As described below, the second color-producing light source 15 enables detection of various types of diseased sites and the like in relation to the filter provided to the wavelength adjusting device 16.

The wavelengths of the first color-producing light source 14 and the second color-producing light source 15 can be seen as follows in relation to the filter. That is, a visible light, ultraviolet light, or near-infrared light may be used as the light source. In the visible light region, a purple light, blue light, and green light can be used to carry out a number of effective inspections.
Using any one of such a visible light, ultraviolet light, and near-infrared light, in combination with the filter, it is possible to detect a light (including fluorescent lights) having a wavelength different from that of the lights emitted from the light source from a site in the enamel and dentin in the oral cavity, the site having early forms of mineral loss from tooth cavity, tooth erosion, and tooth surface porosity, thereby enabling detection of these diseases. Accordingly, it is possible to detect a site in the enamel having mineral loss before becoming hollow, more effectively than conventional visual inspections using only a white light.
Likewise, using any one of a blue light, purple light, and ultraviolet light, in relation to the filter, it is possible to detect, for example, a mild fluorosis, heavily accumulated plaque and dental calculus, undersurface void, surface void and the like. Moreover, it is possible to distinguish a site having a dental repair material formed and a stained tooth. For example, it has conventionally been very difficult to detect restorative materials made of silver amalgam, titanium, and gold; and when the restorative material is a tooth-colored polymer, ceramic and the like, it has been difficult to detect the existence thereof and the boundaries of the dental restorative materials. In the dental technology today, many of the restorative materials that are used have a tooth color in this way; therefore, the present invention, which can identify these restorative materials, is very useful.

To give one example, a blue light, purple light, and ultraviolet light cause a fluorescent light (e.g. red) having a wavelength which is longer than the wavelength of the light from the light source from plaque accumulated on a tooth surface, a restorative material, an artificial tooth, and other dental restorative materials. These can be detected by appropriately separating this fluorescent light with the below-mentioned filter.
Likewise, to give another example, a lesion in the soft tissue also emits a fluorescent light (e.g. green) having a wavelength which is longer than the wavelength of the light from the light source based on a blue light, purple light, or ultraviolet light irradiated thereto. By appropriately separating a fluorescent light produced by keratin in the oral mucosal epithelial cell with the below-described filter, it is possible to detect pathological ranges related to the mucous membrane, such as ranges of ulcer, keratinization, chronic inflammation, and cancer.

As seen from Figs. 1 and 2, the white light sources 13, the first color-producing light sources 14, and the second color-producing light sources 15 are respectively arranged in plural numbers on the bottom part of the hood 12 at certain intervals in a concentric manner around the cylindrical axis of the hood 12. The plurality of light sources are arranged evenly based on the type of color of the light sources used so as to be able to irradiate the oral cavity uniformly. Further, it is preferable to arrange the white light sources 13, the first color-producing light sources 14, and the second color-producing light sources 15 in plural numbers in such a manner that shadowless effects can be attained in order to prevent shadows in the oral cavity.

The light source unit 11 is configured to enable selecting and switching among: a normal observation mode in which the white light sources 13 are turned on and the first color-producing light sources 14 and second color-producing light sources 15 are turned off; a first QLF mode in which the first color-producing light sources 14 are turned on and the white light sources 13 and second color-producing light sources 15 are turned off; and a second QLF mode in which the second color-producing light sources 15 are turned on and the white light sources 13 and first color-producing light sources 14 are turned off. Each of the modes will be described later.
Selections of the light sources (or selections of the modes) may be switched manually as described below or may be switched automatically under predetermined conditions based on the number of times at shots, and so on. Furthermore, the light sources (or the modes) may be switched optionally by a user based on the setting of an intraoral inspection apparatus.

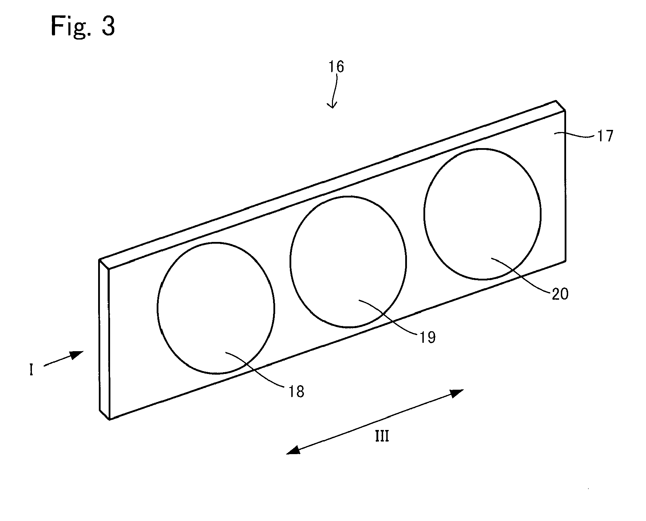

Next, the wavelength adjusting device 16 will be described. Fig. 3 illustrates a perspective view of the wavelength adjusting device 16. The wavelength adjusting device 16 illustrated in Fig. 1 is a view thereof seen in a direction of the arrow I given in Fig. 3. The wavelength adjusting device 16 is a device for blocking a light in a predetermined wavelength range or for attenuating the amount of the light. Specifically, in the present embodiment the wavelength adjusting device 16 comprises: a case 17 which is a board-like member having three holes aligned; and a filter 18 for a white light source, a first filter 19, and a second filter 20 which are fitted in the three holes of the case respectively.

The filter 18 for a white light source is an optical filter to be used when the white light sources 13 are turned on. Specifically, the filter 18 for a white light source may be a filter which transmits almost all lights regardless of the wavelength. Therefore, although in the present embodiment the filter 18 for a white light source is arranged in one of the holes of the case 17, the hole may be left open instead, without arranging anything therein.

The first filter 19 is an optical filter to be used when the first color-producing light sources 14 are turned on. The first filter 19 may be a device for efficiently transmitting, among the lights which try to transmit through the first filter 19, a colored light emitted from diseased sites and the like in the so-called soft tissue having biological organic tissues such as plaque and dental calculus, and blocking a light having a wavelength other than this or attenuating the amount of the light. A specific configuration of the wavelength characteristics of the first filter 19 may be determined such that the functions of the first filter 19 can be attained in combination with the first color-producing light source 14, and thus is not particularly limited.
To give a specific example, when employing a near-ultraviolet light source (having a peak wavelength in a range of 200 nm to 380 nm) as the first color-producing light source and employing a red colored or orange colored filter (a filter which attenuates a wavelength outside 590 nm to 750 nm) as the first filter, it is possible to extract dental calculus accumulated on a tooth at the gingival margin to make it clearly visible in a form of a fluorescent color different from the color in the surrounding sites.

The second filter 20 is an optical filter to be used when the second color-producing light sources 15 are turned on. The second filter 20 is a device for efficiently transmitting, among the lights which try to transmit through the second filter 20, for example a colored light emitted from a site in the hard tissue (enamel and dentin) in the oral cavity having early forms of mineral loss from tooth cavity, tooth erosion, and tooth surface porosity, and blocking a light having a wavelength other than this or attenuating the amount of the light. A specific configuration of the wavelength characteristics of the second filter 20 may be determined such that the functions of the second filter 20 can be attained in combination with the second color-producing light source 15, and thus is not particularly limited.
To give a specific example, when employing a near-ultraviolet light source (having a peak wavelength in a range of 200 nm to 380 nm) as the second color-producing light source and employing a red colored filter (a filter which attenuates a wavelength outside 620 nm to 750 nm) as the second filter, it is possible to darken the site of the mineral loss in the enamel and to make the site of the resin as the dental repair material brighter than the other sites, thereby enabling extraction thereof to make them clearly visible.

Namely, in combination of the wavelength of the light source used, the type of diseased sites and the like irradiated with this light source, and the filter employed, the diseased sites and the like are extracted in a form of having a color different from that of the surrounding sites, in other words, being brighter than the surrounding sites, thereby enabling clearly distinguishing, by the naked eye, the diseased sites and the like thus extracted.

The first filter 19 and the second filter 20 are not particularly limited as long as they can adjust the wavelength in this way; a commonly used optical filter may be used. Further, a surface treatment for preventing fog is preferably given to the face of the first filter 19 or the second filter 20 which surface is on the hood 12 side. This enables prevention of fog caused by the breath or the like from the oral cavity.

The wavelength adjusting device 16 described above is disposed between the light source unit 11 and the imaging device 21 (see Fig. 1), slidably in a direction illustrated by the arrow III in Fig. 3. That is, by sliding the wavelength adjusting device 16, it is possible to select one of the filter 18 for a white light source, the first filter 19, and the second filter 20 to arrange it in a manner to cover up the hole 12a of the hood 12 of the light source unit 11, and possible to adjust the wavelength of the light from the oral cavity which is taken into the imaging device 21. The details will be described later.

In the present embodiment, the case 17 of the wavelength adjusting device 16 is configured to be a board-like member elongated in one direction as illustrated in Fig. 3, and each of the filters is aligned in its longitudinal direction; thereby enabling the case 17 to be slid to select the filter. However, the case 17 is not limited to this configuration; for example, the case may be in a circular board shape and the filters may be arranged concentrically within the circular board. With this configuration, the circular board shaped case is rotated, and thereby the filter can be selected and arranged in a manner to cover up the hole 12a of the hood 12 of the light source unit 11.

Next, the imaging device 21 will be described with reference back to Fig. 1. The imaging device 21 is a device which receives a light from the oral cavity transmitted through the wavelength adjusting device 16 and converts the light into electrical signals pixel by pixel. Namely, as long as the imaging device 21 has a light-receiving element such as CCD and CMOS, it is not particularly limited. Examples of the imaging device 21 include a digital still camera and a digital video camera.

The processing device 22 is a device which gives predetermined processing to the image electrical signals obtained in the imaging device 21 and can output them as an image or motion picture. Therefore, for example, the processing device 22 comprises: an input port to which signals from the imaging device 21 are inputted; a memory device (ROM) in which a predetermined computing equation and necessary information are stored; a central processing unit (CPU) for carrying out an operation; a memory device (RAM) which functions as an operating area and an area for storing temporary information; and an output port for outputting the operation results.

The processing device 22 in the present embodiment is configured to be capable of carrying out processing by switching three kinds of processing corresponding to the following three modes. In specific, a first processing is a white light source processing corresponding to the normal observation mode in which the white light sources 13 are turned on and the filter 18 for a white light source is employed, and the first color-producing light sources 14 and the second color-producing light sources 15 are turned off. With this processing, it is possible to obtain a normal image or motion picture.
A second processing is a first QLF processing corresponding to the first QLF mode in which the first color-producing light sources 14 are turned on and the first filter 19 is employed, and the white light sources 13 and the second color-producing light sources 15 are turned off.
A third processing is a second QLF processing corresponding to the second QLF mode in which the second color-producing light sources 15 are turned on and the second filter 20 is employed, and the white light sources 13 and the first color-producing light sources 14 are turned off.
Each of the modes will be described later together with the functions of the intraoral inspection apparatus 10.

It should be noted that, as described below, since the information from the light received by the imaging device 21 includes enough information to distinguish diseased sites and the like, the processing device 22 is a device for helping the images or motion pictures to be even easier to see.

In the present embodiment, the processing device 22 is incorporated into the imaging device 21. This enables the intraoral inspection apparatus 10 to be compact and easy to use. Note that this is not to exclude a configuration in which the processing device 22 is arranged separately from the imaging device 21, and the processing device 22 may be externally-connected.

Further, the imaging device 21 may be provided with a power source for carrying out switching of the light source unit 11 and of the wavelength adjusting device 16 and operating the imaging device 21 and the processing device 22. With this configuration, portability and handleability of the device can be easily attained.

Next, the functions of the intraoral inspection apparatus 10 will be described with reference to Figs. 1 to 3.

When carrying out normal observations of the oral cavity with the intraoral inspection apparatus 10, the oral cavity is observed by setting the intraoral inspection apparatus 10 in the normal observation mode. At this time, the white light sources 13 are turned on and the filter 18 for a white light source is employed, and the white light source processing is selected in the processing device 22. And at this point, the first color-producing light sources 14 and the second color-producing light sources 15 are turned off.

The light emitted from the white light source 13 is irradiated to the tooth and the gum as illustrated by the arrows Ia and Ia in Fig. 1. The light reflected from the teeth and the gum reaches the imaging device 21 as illustrated by the arrow Ib in Fig. 1. That is, the reflected light passes through the hole 12a of the hood 12 and transmits through the filter 18 for a white light source of the wavelength adjusting device 16. At this point, lights with all wavelengths are transmitted through the filter 18 for a white light source generally as they are. Then, the imaging device 21 receives the transmitted lights and converts them into electrical signals. Thus at this point, the imaging device 21 receives the light irradiated into the oral cavity by the white light source 13, generally as it is. Accordingly, the processing device 22 may output the obtained image or motion picture without making any remarkable modifications. Herein, color modifications may be carried out such as making the color of the gum tissue in the image closer to the color of the actual gum tissue.

When carrying out observations of the oral cavity by means of a first QLF, the first QLF mode is employed. At this time, the first color-producing light sources 14 are turned on and the first filter 19 is employed, and the first QLF processing is selected in the processing device 22. At this point, the white light sources 13 and the second color-producing light sources 15 are turned off.

In the same manner as in the case of the white light source 13, the light emitted from the first color-producing light source 14 is irradiated to the teeth and the gum as illustrated by the arrows Ia and Ia in Fig. 1. In the case of the first QLF mode, because of the characteristics of the light source, for example a color of a fluorescence having a wavelength different from the wavelength of the irradiated light and different from the fluorescence from the hard tissue is produced from diseased sites and the like in the soft tissue (a site in the tooth and gum having an organic tissue such as plaque and dental calculus). Then the light reflected from the teeth and the gum and the fluorescent light from the diseased sites and the like reach the imaging device 21 as illustrated by the arrow Ib in Fig. 1. At this point, the reflected light and the fluorescent light pass through the hole 12a of the hood 12 and transmit through the first filter 19. At this point, at least the light having a wavelength component other than the wavelength of the fluorescent light from the soft tissue is blocked or the amount of the light is attenuated. Herein, the reflected light of the first color-producing light source 14 and the fluorescent light from the hard tissue have a peak intensity in the wavelength in which the first filter 19 blocks lights or attenuates the amount of light, so most of these lights are blocked or the amounts of these lights are attenuated by the first filter 19. On the other hand, the fluorescent light from the diseased sites and the like in the soft tissue has a wavelength different from the wavelength of the first color-producing light source 14 and can be transmitted through the first filter 19; therefore, it is received and detected by the imaging device 21 to be converted into electrical signals.

Herein, even the fluorescent lights from the same soft tissue slightly differ from one another in wavelength and intensity depending on the types of diseases and the like (for example, dental calculus and plaque). Accordingly, the types and states of these diseases can be recognized by a form of differences in brightness, and can also be distinguished more precisely.

In the processing device 22, the first QLF processing corresponding to the first QLF mode is selected to carry out processing of the electrical signals of the received light. In specific, based on the electrical signals of the received light, a site having the fluorescent light emitted is particularly highlighted, and an image or motion picture which enables the site to be visually easy to recognize is formed. At this time, in addition to showing the site clearly, the color tone of the sites other than the site having the fluorescent light emitted may be adjusted to be close to the natural color of the oral cavity. This enables the image and motion picture to be visually easy to understand.

When carrying out observations of the oral cavity by means of a second QLF, the second QLF mode is employed. At this time, the second color-producing light sources 15 are turned on, and the second filter 20 is employed, and the second QLF processing is selected in the processing device 22. At this point, the white light sources 13 and the first color-producing light sources 14 are turned off.

In the same manner as in the case of the white light source 13, the light emitted from the second color-producing light source 15 is irradiated to the teeth and the gum as illustrated by the arrows Ia and Ia in Fig. 1. In the case of the second QLF mode, because of the characteristics of the light source, for example a color of a fluorescence having a wavelength different from the wavelength of the irradiated light and different from the fluorescence from the soft tissue is produced from diseased sites and the like in the hard tissue (a site in which materials having an inorganic tissue such as dental impression materials and dental restorative materials are disposed, normal teeth, etc.). Then, the light reflected from the teeth and the gum and the fluorescent light from the diseased sites and the like reach the imaging device 21 as illustrated by the arrow Ib in Fig. 1. At this point, the reflected light and the fluorescent light pass through the hole 12a of the hood 12 and transmit through the second filter 20. At this point, at least the light having a wavelength component other than the wavelength of the fluorescent light from the hard tissue is blocked or the amount of the light is attenuated. Herein, the reflected light of the second color-producing light source 15 and the fluorescent light from the soft tissue have a peak intensity in the wavelength in which the second filter 20 blocks lights or attenuates the amount of light, so most of these lights are blocked or the amounts of these lights are attenuated by the second filter 20. On the other hand, the fluorescent light from the diseased sites and the like in the hard tissue has a wavelength different from the wavelength of the second color-producing light source 15 and can be transmitted through the second filter 20; therefore, it is received and detected by the imaging device 21 to be converted into electrical signals.

Herein, even the fluorescent lights from the same hard tissue slightly differ from one another in wavelength and intensity depending on the types of diseases and the like (for example, dental impression materials and healthy teeth, and other diseases). Accordingly, the types and states of these diseases can be recognized by a form of differences in brightness, and can also be distinguished more precisely.

In the processing device 22, the second QLF processing corresponding to the second QLF mode is selected to carry out processing of the electrical signals of the received light. In specific, based on the electrical signals of the received light, a site having the fluorescent light emitted is particularly highlighted, and an image or motion picture which enables the site to be visually easy to recognize is formed. At this time, in addition to showing the site clearly, the color tone of the sites other than the site having the fluorescent light emitted may be adjusted to be close to the natural color of the oral cavity. This enables the image and motion picture to be visually easy to understand.

In the present embodiment, for the advantage of being able to obtain an image or motion picture even easier to see, the electrical signals are processed by using the processing device 22. That is, the processing of the electrical signals may be simplified, which is to just highlight the diseased sites and the like. Accordingly, with the intraoral inspection apparatus 10, it is possible to sufficiently distinguish and detect diseased sites and the like based on the information obtained from the light received by the imaging device 21, not requiring complex signal processing. This can also inhibit an error during observation from ocurring.

As described above, according to the intraoral inspection apparatus 10, it is possible to easily distinguish and detect a plurality of diseased sites and the like of more kinds than before with one apparatus. As in the present embodiment, when observations using the white light source can also be carried out, it is possible to carry out the normal oral observations of the oral cavity, enabling easily comparing the images obtained through the normal observations with the images of diseased sites and the like. That is, a plurality of images and motion pictures can be obtained by using a white light and light of other colors; therefore pathological inspections can be enhanced. The QLF combined with images and motion pictures obtained by using a white light makes apparent a site where there is a fear of a pathological lesion of the oral cavity and enables the site to be recognized at an early stage. At this time, the procedure is simple in this way, does not cause pain to a patient, and thus is non-invasive. It can also help provide oral health education for patients and the like.

In switching the normal observation mode, the first QLF mode, and the second QLF mode, it is preferable to be able to select the way to do so, among manually, automatically, and both manually and automatically as needed. By switching the modes manually, the necessary images and motion pictures can be taken adequately. At this time, in view of convenience it is preferable to be able to carry out selections of the light source, the filter, and the processing device with a single switch. Further, for switching automatically, the setting may be given such that the normal observation mode, the first QLF mode, and the second QLF mode are switched automatically in turns every one or two shots. This enables the user to finish shooting in all the modes by shooting at certain number of times without particularly concerning about the present mode.

Further, in the present embodiment, a configuration has been described in which configuration a plurality of different diseased sites and the like can be distinguished and detected by using the two QLF modes. However, the present invention is not limited to this configuration; modes of a light source, filter, and processing device with other features may be further provided as needed. A combination of the color-producing light source and the filter enables more diseased sites and the like to be distinguished precisely. Therefore, more kinds of color-producing light sources and more kinds of filters corresponding to these light sources may be provided than they are in the present embodiment described above. Additionally, in the present embodiment, the modes of the first color-producing light source and the second color-producing light source are selected by turning them on separately; however, a configuration may also be adopted in which the first and second color-producing light sources are turned simultaneously and the filter corresponding thereto is provided, thereby enabling detection of diseases in a wide range. Namely, the third, the fourth, ... QLF modes may also be added.

In the present invention, in carrying out the above-described QLF, dyes such as fluorescein diacetate and sodium fluorochrome may be used. These kinds of dyes color plaque, and enhance detection employing a purple light and ultraviolet light.

### Description of the Reference Numerals

- 10: intraoral inspection apparatus
- 11: light source unit
- 12: hood
- 13: white light source
- 14: color-producing light source
- 15: color-producing light source
- 16: wavelength adjusting device
- 17: case
- 18: filter for white light source
- 19: first filter
- 20: second filter
- 21: imaging device
- 22: processing device

## Claims

1. An intraoral inspection apparatus comprising:
a light source unit which is provided with at least one color-producing light source capable of inducing fluorescent lights from diseased sites by irradiating an oral cavity;
a wavelength adjusting device which is provided with a first filter that transmits, among the fluorescent lights, a light having a wavelength of a fluorescent light from a predetermined diseased site and blocks or attenuates a light having a wavelength other than this wavelength, and provided with a second filter that transmits, among the fluorescent lights, a light having a wavelength of a fluorescent light from a diseased site different from the predetermined diseased site and blocks or attenuates a light having a wavelength other than this wavelength; and
an imaging device which receives the fluorescent light transmitted through the first filter and the fluorescent light transmitted through the second filter, and converts them into electrical signals.

2. The intraoral inspection apparatus according to claim 1, further comprising a white light source(s).

3. The intraoral inspection apparatus according to claim 1 or 2, wherein a first QLF mode and a second QLF mode are switched automatically or manually,
the first QLF mode being a mode in which the color-producing light source (s) is/are turned on and the first filter is disposed such that the light transmitted through the first filter is received by the imaging device, and
the second QLF mode being a mode in which the color-producing light source(s) is/are turned on and the second filter is disposed such that the light transmitted through the second filter is received by the imaging device.

4. A method for operating an intraoral inspection apparatus, wherein the intraoral inspection apparatus according to any one of claims 1 to 3 is used;
the color-producing light source(s) is/are turned on, a light from the color-producing light source (s) is irradiated into the oral cavity, and the light from the oral cavity is received by the imaging device through the first filter;
the color-producing light source(s) is/are turned on, a light from the color-producing light source (s) is irradiated into the oral cavity, and the light from the oral cavity is received by the imaging device through the second filter.

5. A method for operating an intraoral inspection apparatus, wherein the intraoral inspection apparatus according to claim 2 is used;
the color-producing light source(s) is/are turned on, a light from the color-producing light source (s) is irradiated into the oral cavity, and the light from the oral cavity is received by the imaging device through the first filter;
the color-producing light source(s) is/are turned on, a light from the color-producing light source (s) is irradiated into the oral cavity, and the light from the oral cavity is received by the imaging device through the second filter; and
the white light source (s) is/are turned on and a light from the oral cavity is received by the imaging device.
